(19) 

**Europäisches Patentamt**
**European Patent Office**
**Office européen des brevets**

(11) **EP 4 582 469 A1**

(12) **EUROPEAN PATENT APPLICATION**

(43) Date of publication:
**09.07.2025 Bulletin 2025/28**

(21) Application number: **24167745.9**

(22) Date of filing: **28.03.2024**

(51) International Patent Classification (IPC):
**C08J 3/24** *(2006.01)* **C08J 3/12** *(2006.01)*

(52) Cooperative Patent Classification (CPC):
**C08J 3/245; C08J 3/12;** B01J 20/26; C08F 220/06;
C08J 2333/06

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **04.01.2024 TW 113100385**

(71) Applicant: **Formosa Plastics Corporation**
**Kaohsiung City (TW)**

(72) Inventors:
• **LEE, Cheng-Lin**
  **Kaohsiung City (TW)**
• **LIN, Ya-Chi**
  **Kaohsiung City (TW)**
• **LAI, Tai-Hong**
  **Kaohsiung City (TW)**
• **CHEN, Zhong-Yi**
  **Kaohsiung City (TW)**

(74) Representative: **Lang, Christian**
**LangPatent Anwaltskanzlei IP Law Firm**
**Ingolstädter Straße 5**
**80807 München (DE)**

(54) **METHOD OF FABRICATING SUPERABSORBENT POLYMER**

(57) A method of fabricating a superabsorbent polymer is provided. The method includes performing a free radical polymerization reaction to a superabsorbent polymer component, so as to obtain a colloid gel. The colloid gel is cut by using a pulverizer to obtain superabsorbent polymer particles. The pulverizer has a perforated plate with changeable hole diameters, which has a first diameter of an inlet hole greater than a second diameter of an outlet hole. The method further includes performing a surface cross-linking reaction to the superabsorbent polymer particles, so as to obtain the superabsorbent polymer. Therefore, a bulk density, an absorption rate and a liquid permeability of the obtained superabsorbent polymer can be increased.

100

| perform a free radical polymerization reaction to a superabsorbent polymer composition to obtain a colloid gel | 110 |

↓

| shear the colloid gel by using a pulverizer to obtain plural superabsorbent polymer particles | 120 |

↓

| perform a surface crosslinking reaction to the superabsorbent polymer particles to obtain the superabsorbent polymer | 130 |

Fig. 1

EP 4 582 469 A1

**Description**

**BACKGROUND**

Field of Invention

The present invention relates to a method of fabricating a superabsorbent polymer. More particularly, the present invention relates to a method of fabricating a superabsorbent polymer by using a pulverizer having a perforated plate with changeable hole diameters.

Description of Related Art

Superabsorbent polymer (SAP) is one kind of polymers without water solubility, and is widely used in various fields, such as absorbent products of diapers and sanitary napkin, water retaining agent for agricultural or horticultural uses, and industrial water-stopping agents.

Large quantities of monomers and hydrophilic polymers are needed in a fabrication process of the superabsorbent polymer, and a production of polyarylic acid (salt)-based superabsorbent polymers by using acrylic acid and /or its salts is majority in industry. With high performance of the diapers, which is the main application, there's demand for the superabsorbent polymers with more functions (such as high price-performance ratio). Specifically, in addition to basic physical properties such as absorption without pressure and absorption against pressure, various physical properties such as gel strength, water soluble component, moisture content, water absorbing rate, anti-bacterial property, wear resistance, powder flowability, deodorizing property, color resistance, low dust and low residual monomer are required for the superabsorbent polymers. Especially, as hygeined products such as the diapers become thinner, it is further desirable to elevate the absorption rate of the superabsorbent polymers.

Typically, the industrial fabrication method of the superabsorbent polymers powders or granules includes following processes: a polymerization process; a gel shearing (grain refinement) process after or during the polymerization process; a drying process for the grain-refined gel; a shearing process for the dried particles; a screening (grading) process for the sheared particles; and a surface crosslinking process for the superabsorbent polymer particles after screening. In recent fabrication methods of superabsorbent polymers, there's a method simultaneously performing the polymerization process and the gel shearing process by using a polymerization device with a pulverizer. In the aforementioned method, obtained hydrogel is sheared during the polymerization reaction of liquid monomers, and the grain-refined hydrogel is discharged from the polymerization device.

However, dimensions of the gel particles obtained by the above device are about a few millimeters to a few centimeters. Under requirement for increasing absorption rate of the superabsorbent polymers, the aforementioned dimensions of gel particles cannot satisfy the requirement; thus, a gel pulverizer is further required. For example, an intermittent kneader reactor or continuous kneader reactor can be used in a wet pulverization method to fabricate the superabsorbent particles with specific particle size or the smaller gel particles. Nevertheless, the size of the conventional gel pulverizer is too big to apply it in the industrial process.

Accordingly, there is an urgent need to provide a method of forming a superabsorbent polymer to pulverize the gel to have size that fits the requirement during the fabrication process.

**SUMMARY**

An aspect of the present invention provides a method of fabricating a superabsorbent polymer, which increases absorption rate and liquid conductivity of the superabsorbent polymer by using a pulverizer having a perforated plate with changeable hole diameters to shear colloid gels.

According to the aspect of the present invention, the method of fabricating the superabsorbent polymer is provided. The method includes performing a free radical polymerization reaction to a superabsorbent polymer composition, so as to obtain a colloid gel, in which the superabsorbent polymer composition includes an acid group-containing monomer aqueous solution, a polymerization initiator, and a free radical polymerization crosslinking agent; shearing the colloid gel by using a pulverizer to obtain plural superabsorbent polymer particles, in which the pulverizer has the perforated plate with the changeable hole diameters, the perforated plate with changeable hole diameters includes an inlet hole and an outlet hole, the inlet hole has a first diameter, the outlet hole has a second diameter, and the first diameter is greater than the second diameter; and performing a surface crosslinking reaction to the superabsorbent polymer particles, a surface crosslinking agent and a reaction polymer, so as to obtain the superabsorbent polymer.

According to an embodiment of the present invention, the first diameter is in a range of 8 mm to 20 mm.

According to an embodiment of the present invention, the second diameter is in a range of 6 mm to 18 mm.

According to an embodiment of the present invention, a thickness of the perforated plate with changeable hole diameters is in a range of 20 mm to 40 mm.

According to an embodiment of the present invention, the first diameter, the second diameter and the thickness of the perforated plate with changeable hole diameters have following formula:

$$\alpha = \frac{(D1 - D2)/2}{L}$$

in the above formula, D1 represents the first diameter, D2 represents the second diameter, and L represents the thickness of the perforated plate with changeable hole diameters; and a value of $\alpha$ is in a range of 0.05 to 0.35.

According to an embodiment of the present invention, shearing the colloid gel further includes screening plural small colloid gels with an average diameter not greater than 2.00 mm.

According to an embodiment of the present invention, the method further includes adding a surface crosslinking agent and an aluminum salt to the superabsorbent polymer particles before performing the surface crosslinking reaction.

According to an embodiment of the present invention, based on an amount of the superabsorbent polymer particles as 100 wt%, an amount of the aluminum salt is 0.1 wt% to 1.0 wt%.

According to an embodiment of the present invention, the aluminum salt includes aluminum sulfate, aluminum lactate, aluminum citrate and combinations thereof.

According to the aspect of the present invention, providing the method of fabricating the superabsorbent polymer. The method includes performing a free radical polymerization reaction to a superabsorbent polymer composition, so as to obtain a colloid gel, in which the superabsorbent polymer composition includes an unsaturated monomer aqueous solution, a polymerization initiator, and a free radical polymerization crosslinking agent; shearing the colloid gel by using a pulverizer to obtain plural superabsorbent polymer particles, in which the pulverizer has a perforated plate with changeable hole diameters, the perforated plate with changeable hole diameters includes an inlet hole with a first diameter in a range of 8 mm to 20 mm and an outlet hole with a second diameter in a range of 6 mm to 18 mm; and performing a surface crosslinking reaction to the superabsorbent polymer particles and a surface crosslinking agent, so as to obtain the superabsorbent polymer.

According to an embodiment of the present invention, the first diameter is greater than the second diameter.

According to an embodiment of the present invention, a thickness of the perforated plate with changeable hole diameters is in a range of 20 mm to 40 mm.

According to an embodiment of the present invention, the first diameter, the second diameter and the thickness of the perforated plate with changeable hole diameters have following formula:

$$\alpha = \frac{(D1 - D2)/2}{L}$$

in the above formula, D1 represents the first diameter, D2 represents the second diameter, and L represents the thickness of the perforated plate with changeable hole diameters; and a value of $\alpha$ is in a range of 0.05 to 0.35.

According to an embodiment of the present invention, the method further includes adding an aluminum salt before performing the surface crosslinking reaction, wherein based on an amount of the superabsorbent polymer particles as 100 wt%, an amount of the aluminum salt is 0.1 wt% to 1.0 wt%.

According to an embodiment of the present invention, based on an amount of the unsaturated monomer aqueous solution as 100 wt%, an amount of the free radical polymerization crosslinking agent is 0.001 wt% to 5 wt%.

With the application of the superabsorbent polymers and the method of fabricating the same of the present invention, compactness and surface roughness of the colloid gel can be increased by shearing the colloid gel by using the pulverizer having the perforated plate with changeable hole diameters. Furthermore, the bulk density, the absorption rate and the liquid conductivity of the obtained superabsorbent polymer can be increased.

**BRIEF DESCRIPTION OF THE DRAWINGS**

Aspects of the present disclosure are best understood from the following detailed description when read with the accompanying figures. It is noted that, in accordance with the standard practice in the industry, various features are not drawn to scale. In fact, the dimensions of the various features can be arbitrarily increased or reduced for clarity of discussion.

Fig. 1 illustrates a flow chart of a method of fabricating a superabsorbent polymer according to some embodiments of the present invention.

Fig. 2 illustrates a side view of an inlet end and an outlet end of a perforated plate with the changeable hole diameters.

## DETAILED DESCRIPTION

As described above, the present invention provides the method of fabricating the superabsorbent polymer, such that the compactness and the surface roughness of the colloid gel can be increased by shearing the colloid gel by using the pulverizer having the perforated plate with changeable hole diameters. Furthermore, the bulk density, the absorption rate and the liquid conductivity of the obtained superabsorbent polymer can be increased.

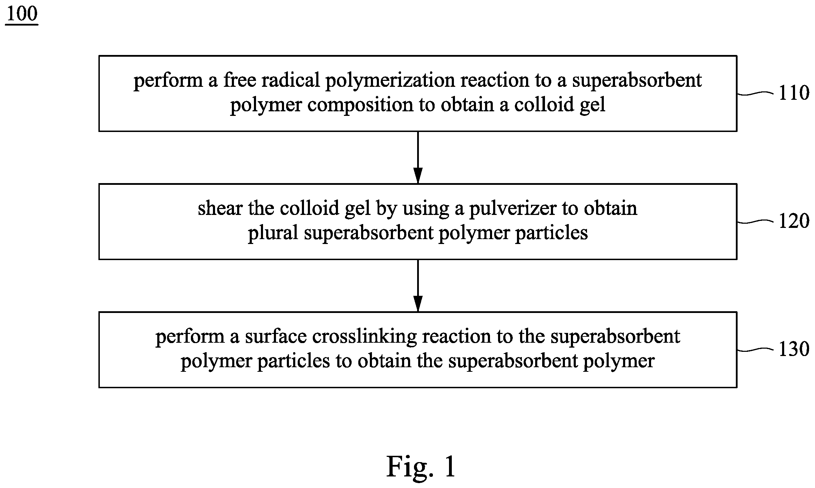

Referring to Fig. 1, Fig. 1 illustrates a flow chart of the method 100 of fabricating the superabsorbent polymer according to some embodiments of the present invention. First, operation 110 is performed to perform a free radical polymerization reaction to a superabsorbent polymer composition, so as to obtain a colloid gel. In some embodiments, the superabsorbent polymer composition includes an acid group-containing monomer aqueous solution, a polymerization initiator, and a free radical polymerization crosslinking agent.

In some embodiments, the acid group-containing monomer aqueous solution of the superabsorbent polymer compositions includes acid group monomers having unsaturated double bonds, such as acrylic acid. In some embodiments, the acid group-containing monomer aqueous solution can be methacrylic acid, 2-acrylamido-2-methylpropanesulfonic acid, maleic acid (cis-butenedioic acid), maleic anhydride, fumaric acid (trans-butenedioic acid), and fumaric acid anhydride. The acid group-containing monomer aqueous solution can include but not limit to one type of monomer, but can also choose two or more aforementioned monomer aqueous solutions.

In some embodiments, based on an amount of the superabsorbent polymer compositions, a concentration of the acid group-containing monomer aqueous solution can be, but not limit to, 20 wt% to 55 wt%, and preferably 30 wt% to 45 wt%. Generally, if the concentration of the acid group-containing monomer aqueous solution is 20 wt% to 55 wt%, viscosity of products after polymerization is suitable; and thus it's easier to perform machining, and reaction heat of free radical polymerization reaction is more controllable.

In some alternative embodiments, other hydrophilic monomers with unsaturated double bonds can be optionally added, such as acrylamide, methacrylamide, 2-carboxyethyl acrylate, 2-carboxyethyl methacrylate, methyl acrylate, ethyl acrylate, dimethylamino acrylamide, and acrylamidopropyltrimonium chloride. However, an adding amount of the hydrophilic monomer is based on a principle of not destroying physical properties (such as centrifuge retention capacity and the absorption rate) of the superabsorbent polymer.

In some embodiments, water-soluble polymers are also optionally added in the superabsorbent polymer composition to reduce processing cost. The water-soluble polymer includes partially saponified or fully saponified polyvinyl alcohol, polyethylene glycol, polyacrylic acid, polyacrylamide, starch or starch derivatives, such as methyl cellulose, acrylate methyl cellulose, ethyl cellulose, and etc., and preferably, the starch and partially saponified or fully saponified polyvinyl alcohols is used individually or are used after mixing. In these embodiments, molecular weight of the water-soluble polymers is not limited. Based on the amount of the acid group-containing monomer aqueous solution as 100 wt%, an adding amount of the water-soluble polymer is on a principle that physical properties of the superabsorbent polymer are not decreased, and generally the adding amount is not greater than 20 wt%, preferably not greater than 10 wt%, and more preferably not greater than 5 wt%.

In some embodiments, the acid group-containing monomer aqueous solution can be directly subjected to the polymerization reaction; or can be firstly partially neutralized with a neutralizing agent to become a neutral solution or a weak acidic solution and then subjected to the polymerization reaction. In these embodiments, the neutralizing agent includes hydroxides or carbonates of alkali metal or alkaline earth metal (e.g. sodium hydroxide, potassium hydroxide, sodium carbonate, potassium carbonate, sodium bicarbonate and potassium bicarbonate), amino compounds or combinations thereof. In some embodiments, a neutralizing concentration of the acid group-containing monomer aqueous solution is 45 mol% to 85 mol%, and preferably 50 mol% to 75 mol%. When the neutralizing concentration is within the aforementioned range, the acid group-containing monomer aqueous solution can have a suitable pH value, and cannot cause damage once contacting with human body carelessly. It is noted that the neutralizing concentration herein is defined as a ratio of mole number of the alkaline solution to mole number of the acid group-containing monomer aqueous solution, or it can also defined as percentage of acid group of the acid group-containing monomer aqueous solution being neutralized. In some embodiments, the pH value of the acid group-containing monomer aqueous solution is 5.5 to 7.0, and preferably 5.5 to 6.5. If the pH value of the acid group-containing monomer aqueous solution is within 5.5 to 7.0, there is less unreacted monomer residues remaining in the solution after polymerization, and the produced superabsorbent polymers can have better physical properties and greater absorption capacity.

Pre-polymerization reaction begins with decomposition of the polymerization initiator to generate free radicals. In some embodiments, based on an amount of the acid group-containing monomer aqueous solution as 100 wt%, a suitable amount of the polymerization initiator is 0.001 wt% to 10 wt%, and preferably 0.1 wt% to 5 wt%. If the amount of the polymerization initiator is within the aforementioned range, a reacting rate of the free radical polymerization reaction is suitable, economic efficiency of the reaction is better, the reaction heat is more controllable, and it can avoid forming solid gel due to excessive polymerization.

In some embodiments, the polymerization initiator includes a thermal decomposition initiator, a redox initiator or combinations thereof. In some embodiments, the thermal decomposition initiator includes peroxide compounds (for example, hydrogen peroxide, di-t-butyl peroxide, peroxyamide or persulfate (including ammonium salt and alkali metal salt)) and azo compounds (for example, 2,2'-azobis(2-amidinopropane) dihydrochloride salt or 2,2'-azobis (N,N-dimethylene isobutylamidine) dihydrochloride salt). In some embodiments, the redox initiator includes bisulfite salt, ascorbic acid or ferrous salt. The polymerization initiators are better used by combining the thermal decomposition initiator and the redox initiator, in which the redox initiator generates free radicals, and when the free radicals transfer to the monomers, the polymerization reaction is induced, and thus great heat energy released from the polymerization reaction increases temperature. When it reaches specific temperature, the thermal decomposition initiator is further decomposed, such that the polymerization reaction can be more completed to avoid remaining excess unreacted monomers.

The free radical polymerization crosslinking agent in the superabsorbent polymer composition makes the superabsorbent polymer composition have suitable degree of crosslinking, thereby increasing processability of the superabsorbent polymer composition after the polymerization. In some embodiments, the free radical polymerization crosslinking agent can select compounds including two or more unsaturated double bonds, such as N,N-bis(2-propenyl) amine, N,N-methylene bisacrylamide, N,N-methylene bismethacrylamide, propylene acrylate, ethylene glycol diacrylate, polyethylene glycol) diacrylate, ethylene glycol dimethacrylate, polyethylene glycol dimethyl acrylate, glycerol triacrylate, glycerol trimethacrylate, glycerol added ethylene oxide triacrylate or trimethacrylate, trimethylolpropane trimethacrylate, trimethylolpropane triacrylate, N,N,N-tris(2-propenyl)amine, diacrylic acid ethylene glycol ester, polyoxyethylene triacrylate glycerol ester, diethylene polyoxyethylene glycerol triacrylate, dipropylene triethylene glycol ester, and etc. In some embodiments, the free radical polymerization crosslinking agent can select compounds having two or more epoxy groups, such as sorbitol polyglycidyl ether, polyglycerol polyglycidyl ether, ethylene glycol diglycidyl ether, diethylene glycol diglycidyl ether, polyethylene glycol diglycidyl ether, diglycerol polyglycidyl ether, and etc. The free radical polymerization crosslinking agent can be used individually, or more than two types of the free radical polymerization crosslinking agent are mixed to be used. In some embodiments, based on the amount of the acid group-containing monomer aqueous solution as 100 wt%, an amount of the free radical polymerization crosslinking agent is 0.001 wt% to 5 wt%, and preferably 0.01 wt% to 3 wt%. If the amount of the free radical polymerization crosslinking agent is within the aforementioned range, viscosity of the polymer solution after reaction is suitable, and thus the polymer solution is easier to be machined. Therefore, the absorption capacity of the produced superabsorbent polymer is better.

In some embodiments, the above free radical polymerization reaction can be performed in a batch reaction vessel or a conveyor reactor.

Subsequently, operation 120 is performed to shear the colloid gel by using a pulverizer, so as to obtain plural superabsorbent polymer particles. The pulverizer has a perforated plate with changeable hole diameters. The pulverizer having the perforated plate with changeable hole diameters is used to decrease number of times of shearing the colloid gel, the colloid gel after shearing can be agglomerated, and the obtained superabsorbent polymer can be firmer, thus reducing water soluble component within the superabsorbent polymer particles, and increasing the bulk density of the superabsorbent polymer particles. In addition, the surface roughness of the colloid gel can be increased, thereby increasing a surface porosity and the absorption rate of the superabsorbent polymer particles, and further increasing the liquid conductivity.

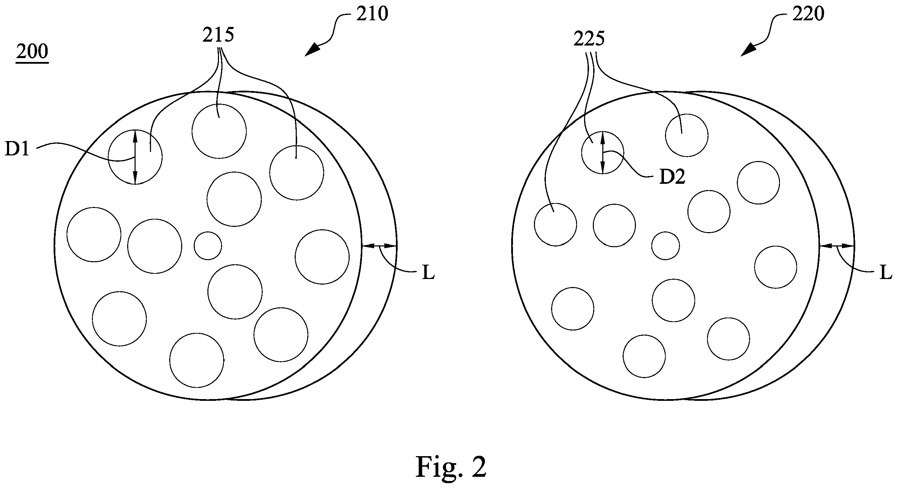

Referring to Fig. 2, Fig. 2 illustrates a side view of an inlet end 210 and an outlet end 220 of the perforated plate with the changeable hole diameters 200. The inlet end 210 includes several inlet holes 215, and the outlet end 220 includes several outlet holes 225. The inlet holes 215 have a first diameter D1, while the outlet holes 225 have a second diameter D2. In some embodiments, the first diameter D1 is greater than the second diameter D2, such that the obtained superabsorbent polymer particles can have better adhesive effect.

In some embodiments, the first diameter D1 is in a range of about 8 mm to about 20 mm, and preferably about 10 mm to about 20 mm. When the first diameter D1 is within the aforementioned range, the equipment (i.e. the pulverizer) can be operated more smoothly, and friction between the colloid gels is increased to increase the surface roughness of the colloid gel, thereby increasing the bulk density and the absorption rate of the obtained superabsorbent polymer particles. In some embodiments, the second diameter D2 is in a range of about 6 mm to about 18 mm, and preferably about 8 mm to about 16 mm. When the second diameter D2 is within the aforementioned range, the equipment (i.e. the pulverizer) can be operated more smoothly, and the obtained superabsorbent polymer particles can have better adhesive effect.

The perforated plate with the changeable hole diameters 200 has a thickness L. In some embodiments, the thickness L is in a range of about 20 mm to about 40 mm, and preferably 30 mm. When the thickness L is within the aforementioned range, the obtained superabsorbent polymer particles can have better adhesive effect, and have suitable discharging rate.

Additionally, the first diameter D1, the second diameter D2 and the thickness L of the perforated plate with the changeable hole diameters 200 have the following formula:

$$\alpha = \frac{(D1 - D2)/2}{L}$$

In some embodiments, a value of $\alpha$ is about 0.05 to about 0.35, and preferably 0.051 to about 0.349. When the value of $\alpha$ is within the aforementioned range, the surface roughness of the colloid gel can be increased, and the obtained superabsorbent polymer particles can have better adhesive effect, thereby becoming firmer. Moreover, the water soluble component within the superabsorbent polymer particles can be reduced, and the bulk density of the obtained superabsorbent polymer particles can be increased.

In some embodiments, steps of drying, shearing and screening should be performed on the obtained small colloid gel after shearing by the pulverizer. In the aforementioned embodiments, temperature of the drying step is about 100°C to about 250°C. When the aforementioned temperature range is used to perform the drying step is used, drying time can be effectively controlled, and degree of crosslinking can be effectively controlled to avoid remaining great amount of unreacted monomers.

In some embodiments, the screening step filters the small colloid gel with the average particle size not greater than about 2.0 mm, and preferably about 0.05 mm to about 1.50 mm. The colloid gel with the average particle size greater than 2.0 mm should be returned to the pulverizer and be sheared again. The particle size should be controlled in the aforementioned range in order to avoid generating more fine powders in back-end process. Thus, the small colloid gel can have better thermal conductivity and avoid great amount of unreacted monomer residues. Generally, while the particle size distribution of the small colloid gel is narrower, the physical properties of the superabsorbent polymers are better, and the drying time and temperature can be effectively controlled.

In some embodiments, after the screening steps, the drying step is optionally performed on the small colloid gel again. In the aforementioned embodiments, the drying process is performed at temperature of about 100°C to about 180°C. The drying process performed in the aforementioned temperature range and can effectively control the degree of crosslinking to avoid great amount of unreacted monomer residues.

In some embodiments, the particle size of the superabsorbent polymer particles is screened to 0.06 mm to 1.00 mm, and preferably 0.10 mm to 0.85 mm. When the particle size of the superabsorbent polymer particles is controlled within the aforementioned range, amount of the fine particles of the product can be decreased, and the absorptivity of the superabsorbent polymer can be better.

In some embodiments, the obtained superabsorbent polymer particles have the centrifuge retention capacity of about 34.0 g/g to about 35.0 g/g, the clean water absorption rate for 1 minute of about 130 g/g to about 160 g/g, the water soluble component for 1 hour of about 4.7% to about 6.0%, the surface porosity of about 0.033c.c./g to about 0.045 c.c./g, the bulk density of about 600 g/L to about 640 g/L, and the free swell rate of about 0.35 g/g/s to about 0.53 g/g/s.

Then, operation 130 is performed to perform a surface crosslinking reaction to the superabsorbent polymer particles, so as to obtain the superabsorbent polymer. Since the superabsorbent polymer is an insolvable hydrophilic polymer, whose interior has a uniform crosslinking structure; typically, crosslinking is further performed at a surface of the superabsorbent polymer to increase the absorption rate, strength of colloids, an anticaking property, the permeability for the liquid, and etc. The surface crosslinking reaction is performed with a surface crosslinking agent having functional groups, which can react with acid groups. In some embodiments, the surface crosslinking agent includes polyol, polyamine, a compound having two or more epoxy groups and alkylene carbonate. For example, the polyol can be glycerol, ethylene glycol, diethylene glycol, triethylene glycol, polyethylene glycol, propylene glycol; the polyamine can be ethylenediamine, diethylenediamine and triethylenediamine; the compound having at least two epoxy groups can be sorbitol polyglycidyl ether, polyglycerol polyglycidyl ether, ethylene glycol diglycidyl ether, diethylene glycol diglycidyl ether, diethylene glycol diglycidyl ether and diglycerol polyglycidyl ether; and the alkylene carbonate can be ethylene glycol carbonate, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 1,3-dioxan-2-one, 4,6-dimethyl-1,3-dioxan-2-one and 1,3-dioxepan-2-one. The surface crosslinking agents can be used individually or combined two or more to perform the reaction. In addition, according to the selected surface crosslinking agent, it can be added directly, or it can be initially prepared as an aqueous solution or a hydrophilic organic solution and then added. The hydrophilic organic solvent includes, but not limit to methanol, ethanol, propanol, isobutanol, acetone, methyl ether, ethyl ether, and etc.

In some embodiments, based on the total solid amount of the reactant as 100 wt%, an adding amount of the surface crosslinking agent is about 0.001 wt% to about 10 wt%, and preferably about 0.005 wt% to about 5 wt%. When the adding amount of the surface crosslinking agent is within the aforementioned range, it subjects the surface of the superabsorbent polymer to have a crosslinking structure, and the absorptivity is further improved.

In some embodiments, the surface crosslinking reaction further includes adding an aluminum salt while adding the surface crosslinking agent, thereby further increasing liquid conductivity of the superabsorbent polymer. In some examples, the aluminum salt includes aluminum sulfate, aluminum lactate, aluminum citrate and combinations thereof. In some embodiments, based on the amount of the superabsorbent polymer particles as 100 wt%, an amount of the aluminum salt is 0.1 wt% to 1.0 wt%, and preferably 0.3 wt% to 0.7 wt%. The aforementioned amount range of the aluminum salt can increase the absorption against pressure and liquid conductivity of the superabsorbent polymers.

As discussed above, the superabsorbent polymers produced by implementing the method 100 of fabricating the superabsorbent polymer have greater bulk density, lower water soluble component and greater surface porosity. In some embodiments, the superabsorbent polymers of the present invention has the bulk density of about 600 g/L to about 650 g/L, the water soluble component for 16 hours of about 5.9% to 8.6%, and the surface porosity of about 0.033 c.c./g to about 0.045 c.c./g.

Moreover, the superabsorbent polymers should have excellent centrifuge retention capacity (CRC) and absorption against pressure (AAP); thus, the superabsorbent polymers would not be broken or the ability of absorbing liquid of the superabsorbent polymers would not be affected due to external pressure exerted on the absorber after the superabsorbent polymer absorbs the liquid. In some embodiments, the centrifuge retention capacity of the superabsorbent polymers of the present invention is not less than 25 g/g, and preferably about 28.0 g/g to about 29.0 g/g. In some embodiments, the absorption against pressure of the superabsorbent polymers of the present invention is greater than 23 g/g, and preferably about 25 g/g to about 26 g/g.

The superabsorbent polymers produced by method 100 can have better absorption rate, which can be evaluated by using free swell rate (FSR). The superabsorbent polymers of the present invention is not less than 0.35 g/g/s, and preferably about 0.35 g/g/s to about 0.55 g/g/s. It is noted that the superabsorbent polymers with greater free swell rate can absorb liquid rapidly without applying pressure.

Liquid absorption capacity of the dry superabsorbent polymers can be assessed by the time of initial fluid uptake of the dry superabsorbent polymers once contacting with the fluid and given as T20 value. A lower T20 value of the superabsorbent polymers means a shorter uptake time that the dry superabsorbent polymers can absorb liquid quickly. T20 value of the superabsorbent polymers of the present invention is not greater than about 150 seconds, such as about 100 seconds to about 150 seconds. It is noted that T20 value is defined as the time for 1 g superabsorbent polymers to absorb 20 g normal saline and 0.01 wt% alcohol ethoxylate compound solution under a pressure of 0.3 psi, in which the alcohol ethoxylate compound has 12 to 14 carbon atoms.

Permeability of the superabsorbent polymers can be assessed by using urine permeability measurement (UPM). The urine permeability measurement is generally used to measure resistance to flow of the pre-swelling layer of the superabsorbent polymers. Therefore, when the superabsorbent polymers have been wetted by the liquid, the superabsorbent polymers with great UPM shows greater permeability. The UPM value of the superabsorbent polymers of the present invention is about $45 \times 10^{-7}$ cm$^3$-s/g to about $65 \times 10^{-7}$ cm$^3$-s/g.

In addition, the liquid conductivity of the superabsorbent polymers can be assessed by fixed height absorption (FHA) and free swell gel bed permeability (free swell GBP). The FHA value measures amount of fluid absorbed by the superabsorbent polymers when the fluid is pulled against gravity over a specific height. In some embodiments, the FHA value of the superabsorbent polymers of the present invention is about 25.3 g/g to about 35.0 g/g. The free swell gel bed permeability is used to evaluate the permeability of expanded base of the superabsorbent polymers. It is understood that said "free swell" condition represents the increase in volume of the superabsorbent polymers are allowed to swell without a limiting load when absorbing the fluid. In some embodiments, the free swell gel bed permeability of the superabsorbent polymers produced by method 100 is about $22.7 \times 10^{-9}$ cm$^2$ to about $32.5 \times 10^{-9}$ cm$^2$.

The following embodiments are provided to better elucidate the practice of the present invention and should not be interpreted in anyway as to limit the scope of same. Those skilled in the art will recognize that various modifications can be made while not departing from the spirit and scope of the invention. All publication and patent applications mentioned in the specification are indicative of the level of those skilled in the art to which this invention pertains.

Production of superabsorbent polymers

Preparation example

437.5 g of sodium hydroxide solution with a concentration of 48 wt% was slowly added into a 2000 c.c. Erlenmeyer flask containing 540 g of acrylic acid and 583.2 g of water, in which adding ratio of sodium hydroxide/acrylic acid was within a range of 0.85 to 0.95, adding time was 2 hours, and a temperature of neutral reaction system in the flask was kept within a range of 15°C to 40°C, thereby obtaining a monomer aqueous solution with monomer concentration of 42 wt%, in which 70 mol% acrylic acid was partially neutralized to sodium acrylate. The monomer aqueous solution was added into a tank reactor with 2L-volume (produced by CHIN LEI MECHANISM CO., LTD.).

Subsequently, 0.9 g of N,N'-methylene bisacrylamide (as free radical polymerization reaction crosslinking agent) was added into the acid group-containing monomer aqueous solution, and the temperature was maintained at about 20°C. Nitrogen was injected through pipeline to perform deoxygenation for 30 minutes.

Afterwards, 0.3 g of hydrogen peroxide, 4.15 g of sodium bisulfite, 23.4 g of sodium carbonate foaming agent with a concentration of 10% and 3.6 g of ammonium persulfate were added as polymerization initiator to perform the free radical polymerization reaction. The colloid gel was obtained after standing still for 30 minutes.

Embodiment 1

The colloid gel of the preparation example was sheared by a pulverizer (#200 produced by SUN ROLLING CORPORATION), in which the pulverizer had a perforated plate with changeable hole diameters (produced by CHIN LEI MECHANISM CO., LTD.). The perforated plate with changeable hole diameters had an inlet hole with a first diameter D1 of 10 mm and an outlet hole with a second diameter D2 of 8 mm, and the thickness L of the perforated plate was 20 mm. The $\alpha$ value calculated according to the above formula was 0.05.

The colloid gel with a particle size of 2 mm and less was screened. Subsequently, a drying process was performed at the temperature of 130°C for 2 hours, and a sieve with a fixed particle size of 0.1 mm to 0.85 mm was used to filter, thus obtaining superabsorbent polymer particles.

Then, 100 g of the superabsorbent polymer particles were weighed and 5 g of an aqueous solution mixed with ethylene glycol, 1,4-butanediol (produced by Formosa Plastics Corporation) and methanol in a volume ratio of 1:1:0.5 as the surface crosslinking agent, and the thermal treatment was performed at a temperature of 200°C for 1 hour. Thus, the superabsorbent polymer was obtained after cooling.

Embodiments 2 to 6

The superabsorbent polymers of embodiments 2 to 6 were produced by using the similar processing step as embodiment 1. The differences were the first diameter D1 of the inlet hole, the second diameter D2 of the outlet hole and the thickness of the perforated plate with changeable hole diameters, and the calculated $\alpha$ value. The first diameter D1 of the inlet hole, the second diameter D2 of the outlet hole and the thickness of embodiments 2-24 are shown in Table 1.

Comparative example 1

According to methods of China patent CN 1206365A, 83.2 part of acrylic acid, 8 parts of sodium acrylate with 37 wt%, 5.5 parts of polyethylene glycol) diacrylate (average moles of ethylene oxide was 8) and 654.5 parts deionized water were mixed to prepare a monomer solution. In the monomer solution, a neutralized ratio of the acrylic acid was 85%, and the monomer concentration was 30%. Nitrogen gas was blown into the monomer solution to remove dissolved oxygen in the monomer solution, while the temperature of the monomer solution was maintain at 24°C.

Subsequently, 77 parts of 2,2'-azobis(2-methylpropionamidine) dihydrochloride in 10 wt% was added, and the monomer solution was stirred simultaneously. After stirring for 3 minutes, the monomer solution including 2,2'-azobis(2-methylpropionamidine) dihydrochloride became white haze, and produced white solid particles with average particle size of about 9 $\mu$m. The solid particles were 2,2'-azobis(2-methylpropionamidine) diacrylates as the foaming agent. After stirring for 5 minutes, 10.8 parts of sodium persulfate solution in 10 wt% and 0.5 part of L-ascorbic acid solution in 1 wt% were added as the free radical polymerization initiator under nitrogen gas, while stirring the monomer solution. After fully stirring, the monomer solution was stood still. After adding the sodium persulfate solution in 10 wt% and L-ascorbic acid solution in 1 wt% for 3 minutes, the polymerization reaction was initiated. The polymerization was performed in a hot water bath, and the temperature of the hot water bath was controlled with increasing temperature of the monomer solution. After adding the sodium persulfate solution in 10 wt% into the monomer solution for 26 minutes, the temperature of the monomer solution reached 97°C. Then, the monomer solution was stood still for 20 minutes, and the temperature of the monomer solution was kept in a range between 70°C and 90°C, thereby performing the polymerization reaction of the acrylate monomer completely. Therefore, a crosslinking colloid gel polymer with bubble (or abbreviated as colloid gel (A) thereafter) was obtained as crosslinked porous polymer.

The obtained colloid gel (A) was continuously pulverized by a rotating disintegrator shown in China patent number CN 1206365A, which was herein incorporated by reference. During the pulverization, the average operation time or the pulverizing time for the colloid gel (A) in the rotating disintegrator was about 0.25 minute. The gel particles obtained through pulverizing the colloid gel (A) had a particles size in a range between about 1 mm to 15 mm. The pulverized gel particles were dried at 160°C by a rotary hot-air dryer for 1 hour. Subsequently, the dried gel particles were sheared by a roller mill, and were screened according to JIS standard. Thus, the particles passed through sieve pores with 850 $\mu$m but not through sieve pores with 150 $\mu$m were obtained as the superabsorbent polymer particles.

Then, a second crosslinking treatment agent was coated to perform the surface crosslinking reaction, thereby obtaining the superabsorbent polymer. Specifically, through mixing 100 parts of the superabsorbent polymer particles and the second crosslinking treatment agent, and heating the obtained mixture at 195°C for 30 minutes, the superabsorbent polymer was obtained. The second crosslinking treatment agent was formed by the following composition: mixing 0.05 part of ethylene glycol diglycidyl ether, 0.5 part of lactic acid, 0.02 part of polyoxyethylene sorbitan monostearate, 0.75 part of isopropanol and 3 parts of water.

Comparative examples 2 to 8

The superabsorbent polymers of comparative examples 2 to 8 were produced by using the similar processing step as embodiment 1. The differences were the first diameter D1 of the inlet hole, the second diameter D2 of the outlet hole and the thickness of the perforated plate with changeable hole diameters, and the calculated $\alpha$ value. The first diameter D1 of the inlet hole, the second diameter D2 of the outlet hole and the thickness of comparative examples 2 to 8 are shown in Table 1.

Evaluation method

In order to evaluate properties of the superabsorbent polymers of the present invention, the following test methods were used to analyze their physical properties. The evaluation was performed at room temperature ($23\pm2°C$) and relative air humidity of $45\pm10\%$ unless otherwise specified. The superabsorbent polymers should be completely mixed before analyzing.

Centrifuge Retention Capacity

Centrifuge Retention Capacity (CRC) was measured in accordance with ERT 241.2(12) test method regulated by European Disposables and Nonwovens Association (EDANA). The Centrifuge Retention Capacity test results of the superabsorbent polymer particles and the superabsorbent polymers are shown in table 2 and table 3, respectively.

Clean water absorption rate for 1 minute

Clean water absorption rate for 1 minute was measured in accordance with ERT 240.2 (12) test method regulated by EDANA, in which the saline solution was replaced by deionized water (clean water), and absorption time was modified to 1 minute from 30 minutes. The clean water absorption rate for 1 minute test results of the superabsorbent polymer particles are shown in table 2.

Bulk density

Bulk density was measured in accordance with ERT 251.0 (12) test method regulated by EDANA. The bulk density test results of the superabsorbent polymer particles and the superabsorbent polymers are shown in table 2 and table 3, respectively.

Surface porosity

Surface porosity was measured by a mercury porosimeter (micromeritics AutoPore® IV 9520), in which a standard filling pressure was about 4 kPa. The surface porosity test results of the superabsorbent polymer particles and the superabsorbent polymers are shown in table 2 and table 3, respectively.

Free swell rate

Free swell rate (FSR, unit: g/g/s) was measured and calculated according to a method described in Patent WO 2012/174026A1. 4 g of the superabsorbent polymer was dried at temperature of $23\pm2°C$ and under a pressure not greater than 0.01 torr for 48 hours, and then about 1 g of the superabsorbent polymer was placed into a beaker and dispersed at bottom of the beaker. Subsequently, 20 g of sodium chloride solution with concentration of 0.9 wt% was poured in. Time required from the liquid once contacted with the superabsorbent polymers to be fully absorbed by the superabsorbent polymer was measured. The free swell rate was amount of the liquid divided by weight of the superabsorbent polymers, and divided by the time required. Average values of 3 repeated test results of the superabsorbent polymer particles and the superabsorbent polymers are shown in table 2 and table 3, respectively.

Water soluble component for 1 hour and 16 hours

Water soluble component for 1 hour and 16 hours were measured in accordance with ERT 470.2 (02) test method regulated by EDANA. The water soluble component for 1 hour test results of the superabsorbent polymer particles and the water soluble component for 16 hours test results of the superabsorbent polymers are shown in table 2 and table 3, respectively.

Absorption against pressure

Absorption against pressure (AAP) was measured in accordance with ERT 442.3(10) test method regulated by EDANA. The absorption against pressure of the superabsorbent polymers for 0.9% of sodium chloride solution was tested under pressure of 4.9 kPa for 60 minutes. The test results of the superabsorbent polymers are shown in table 3.

T20 value

T20 value (unit: seconds) was measured and calculated according to a method described in U.S. Patent Publication No. 9,285,302. T20 value is defined as time of 1 g superabsorbent polymer required to absorb 20 g normal saline and 0.01 wt% alcohol ethoxylate compound solution, in which the alcohol ethoxylate compound has 12 to 14 carbon atoms. Average values of tests repeated three times of the superabsorbent polymers are shown in table 3.

Urine permeability measurement

Urine permeability measurement (UPM) was measured and calculated according to a method described in Patent WO 2012/174026A1. The test results of the superabsorbent polymers are shown in table 3.

Fixed height absorption

Fixed height absorption (FHA) was measured and calculated according to a method described in U.S. Patent Publication No. 7,108,916. The test results of the superabsorbent polymers are shown in table 3.

Free swell gel bed permeability

Free swell gel bed permeability was measured and calculated according to a method described in U.S. Patent Publication No. 8,021,998B2. The test results of the superabsorbent polymers are shown in table 3.

Table 1

| | thickness of the perforated plate L (mm) | first diameter D1 (mm) | second diameter D2 (mm) | α value |
|---|---|---|---|---|
| Embodiment 1 | 20 | 10 | 8 | 0.050 |
| Embodiment 2 | 30 | 10 | 8 | 0.033 |
| Embodiment 3 | 40 | 10 | 8 | 0.025 |
| Embodiment 4 | 20 | 10 | 6 | 0.100 |
| Embodiment 5 | 30 | 10 | 6 | 0.067 |
| Embodiment 6 | 40 | 10 | 6 | 0.050 |
| Embodiment 7 | 20 | 16 | 12 | 0.100 |
| Embodiment 8 | 30 | 16 | 12 | 0.067 |
| Embodiment 9 | 40 | 16 | 12 | 0.050 |
| Embodiment 10 | 20 | 16 | 8 | 0.200 |
| Embodiment 11 | 30 | 16 | 8 | 0.133 |
| Embodiment 12 | 40 | 16 | 8 | 0.100 |
| Embodiment 13 | 20 | 16 | 6 | 0.250 |
| Embodiment 14 | 30 | 16 | 6 | 0.167 |
| Embodiment 15 | 40 | 16 | 6 | 0.125 |
| Embodiment 16 | 20 | 20 | 16 | 0.100 |
| Embodiment 17 | 30 | 20 | 16 | 0.067 |
| Embodiment 18 | 40 | 20 | 16 | 0.050 |
| Embodiment 19 | 20 | 20 | 8 | 0.300 |

(continued)

| | thickness of the perforated plate L (mm) | first diameter D1 (mm) | second diameter D2 (mm) | α value |
|---|---|---|---|---|
| Embodiment 20 | 30 | 20 | 8 | 0.200 |
| Embodiment 21 | 40 | 20 | 8 | 0.150 |
| Embodiment 22 | 20 | 20 | 6 | 0.350 |
| Embodiment 23 | 30 | 20 | 6 | 0.233 |
| Embodiment 24 | 40 | 20 | 6 | 0.175 |
| Comparative example 1 | - | - | - | - |
| Comparative example 2 | 15 | 10 | 8 | 0.067 |
| Comparative example 3 | 45 | 10 | 8 | 0.022 |
| Comparative example 4 | 20 | 10 | 4 | 0.150 |
| Comparative example 5 | 20 | 22 | 8 | 0.350 |
| Comparative example 6 | 40 | 22 | 8 | 0.175 |
| Comparative example 7 | 30 | 10 | 10 | 0 |
| Comparative example 8 | 30 | 20 | 20 | 0 |

Table 2

| | CRC (g/g) | Clean water absorption rate for 1 minute (g/g) | Water extractable content for 1 hour (%) | Surface porosity (c.c/g) | Bulk density (g/L) | Free swell rate (g/g/s) |
|---|---|---|---|---|---|---|
| Embodiment 1 | 35.2 | 152 | 5.9 | 0.044 | 632 | 0.51 |
| Embodiment 2 | 34.8 | 149 | 5.1 | 0.042 | 630 | 0.49 |
| Embodiment 3 | 35.1 | 147 | 5.7 | 0.041 | 633 | 0.48 |
| Embodiment 4 | 34.6 | 155 | 5.3 | 0.045 | 636 | 0.52 |
| Embodiment 5 | 35.3 | 149 | 5.7 | 0.043 | 639 | 0.50 |
| Embodiment 6 | 35.1 | 147 | 5.6 | 0.041 | 640 | 0.47 |
| Embodiment 7 | 35.4 | 147 | 5.8 | 0.042 | 614 | 0.48 |
| Embodiment 8 | 34.9 | 142 | 4.9 | 0.040 | 616 | 0.45 |
| Embodiment 9 | 35.2 | 140 | 5.8 | 0.039 | 617 | 0.44 |
| Embodiment 10 | 35.6 | 138 | 5.7 | 0.038 | 621 | 0.41 |
| Embodiment 11 | 35.1 | 140 | 5.2 | 0.038 | 623 | 0.42 |
| Embodiment 12 | 34.9 | 144 | 5.1 | 0.040 | 625 | 0.44 |
| Embodiment 13 | 34.8 | 138 | 4.8 | 0.038 | 627 | 0.41 |
| Embodiment 14 | 35.2 | 136 | 5.5 | 0.036 | 628 | 0.38 |
| Embodiment 15 | 35.1 | 139 | 5.4 | 0.039 | 630 | 0.41 |
| Embodiment 16 | 34.7 | 132 | 4.8 | 0.035 | 608 | 0.37 |
| Embodiment 17 | 34.9 | 139 | 4.9 | 0.039 | 610 | 0.41 |
| Embodiment 18 | 35.3 | 137 | 5.8 | 0.038 | 612 | 0.41 |
| Embodiment 19 | 35.4 | 142 | 5.7 | 0.040 | 602 | 0.43 |
| Embodiment 20 | 34.9 | 144 | 4.8 | 0.042 | 605 | 0.47 |
| Embodiment 21 | 35.1 | 140 | 5.3 | 0.037 | 603 | 0.40 |

(continued)

|  | CRC (g/g) | Clean water absorption rate for 1 minute (g/g) | Water extractable content for 1 hour (%) | Surface porosity (c.c/g) | Bulk density (g/L) | Free swell rate (g/g/s) |
|---|---|---|---|---|---|---|
| Embodiment 22 | 34.7 | 134 | 5.1 | 0.034 | 609 | 0.36 |
| Embodiment 23 | 35.3 | 136 | 5.7 | 0.035 | 607 | 0.37 |
| Embodiment 24 | 35.1 | 138 | 5.3 | 0.037 | 610 | 0.40 |
| Comparative example 1 | 45.5 | 98 | 8.2 | 0.025 | 608 | 0.22 |
| Comparative example 2 | 36.4 | 110 | 8.5 | 0.029 | 618 | 0.29 |
| Comparative example 3 | 35.1 | 122 | 5.7 | 0.035 | 621 | 0.39 |
| Comparative example 4 | - | - | - | - | - | - |
| Comparative example 5 | 34.3 | 125 | 6.3 | 0.032 | 630 | 0.33 |
| Comparative example 6 | 35.1 | 120 | 6.1 | 0.031 | 627 | 0.32 |
| Comparative example 7 | 35.3 | 123 | 10.3 | 0.032 | 622 | 0.31 |
| Comparative example 8 | 34.2 | 112 | 7.1 | 0.028 | 612 | 0.30 |

Table 3

|  | CRC (g/g) | Absorption against pressure (g/g) | UPM ($10^{-7}$ cm$^3$s/g) | Surface porosity (c.c/g) | T20 (sec) | Free swell rate (g/g/s) | Water extractable content for 16 hours (%) | Bulk density (g/L) | FHA (g/g) | Free swell GBP ($\times 10^{-9}$ cm$^2$) |
|---|---|---|---|---|---|---|---|---|---|---|
| Embodiment 1 | 28.9 | 25.6 | 45 | 0.045 | 105 | 0.50 | 6.9 | 635 | 29.9 | 32.5 |
| Embodiment 2 | 28.5 | 26 | 50 | 0.043 | 100 | 0.49 | 6.1 | 632 | 29.3 | 32.4 |
| Embodiment 3 | 28.6 | 25.7 | 49 | 0.040 | 108 | 0.47 | 6.6 | 633 | 29.4 | 32.3 |
| Embodiment 4 | 28.4 | 25.4 | 52 | 0.044 | 112 | 0.51 | 6.4 | 638 | 28.7 | 29.9 |
| Embodiment 5 | 28.5 | 25.6 | 53 | 0.044 | 110 | 0.50 | 6.7 | 640 | 28.6 | 29.7 |
| Embodiment 6 | 28.3 | 25.5 | 55 | 0.042 | 115 | 0.47 | 6.5 | 642 | 28.6 | 29.6 |
| Embodiment 7 | 28.7 | 25.3 | 49 | 0.041 | 120 | 0.47 | 6.9 | 616 | 25.6 | 27.9 |
| Embodiment 8 | 28.1 | 25.7 | 61 | 0.041 | 118 | 0.45 | 5.9 | 618 | 25.7 | 28.7 |
| Embodiment 9 | 28.5 | 25.6 | 52 | 0.039 | 122 | 0.42 | 6.8 | 618 | 25.4 | 28.3 |
| Embodiment 10 | 28.4 | 25.3 | 53 | 0.037 | 130 | 0.41 | 6.7 | 623 | 27.9 | 27.1 |
| Embodiment 11 | 28.6 | 25.7 | 48 | 0.038 | 128 | 0.41 | 6.3 | 625 | 27.6 | 26.8 |
| Embodiment 12 | 28.3 | 25.8 | 57 | 0.041 | 129 | 0.44 | 6.1 | 629 | 27.4 | 26.8 |

(continued)

| | CRC (g/g) | Absorption against pressure (g/g) | UPM ($10^{-7}$ cm$^3$s/g) | Surface porosity (c.c/g) | T20 (sec) | Free swell rate (g/g/s) | Water extractable content for 16 hours (%) | Bulk density (g/L) | FHA (g/g) | Free swell GBP ($\times 10^{-9}$ cm$^2$) |
|---|---|---|---|---|---|---|---|---|---|---|
| Embodiment 13 | 28.6 | 25.5 | 53 | 0.038 | 127 | 0.41 | 5.9 | 629 | 28.1 | 26.7 |
| Embodiment 14 | 28.9 | 25.6 | 47 | 0.035 | 125 | 0.38 | 6.6 | 630 | 28.1 | 26.4 |
| Embodiment 15 | 28.7 | 25.4 | 49 | 0.039 | 123 | 0.40 | 6.5 | 632 | 28.3 | 26.3 |
| Embodiment 16 | 28.6 | 25.6 | 50 | 0.036 | 148 | 0.37 | 5.9 | 609 | 27.4 | 25.4 |
| Embodiment 17 | 28.5 | 25.7 | 55 | 0.038 | 145 | 0.40 | 6.1 | 612 | 27.5 | 25.5 |
| Embodiment 18 | 28.7 | 25.6 | 51 | 0.038 | 143 | 0.41 | 7.1 | 613 | 27.3 | 25.1 |
| Embodiment 19 | 28.6 | 25.7 | 50 | 0.040 | 139 | 0.42 | 6.7 | 605 | 26.5 | 24.2 |
| Embodiment 20 | 28.3 | 25.5 | 58 | 0.041 | 137 | 0.45 | 5.8 | 607 | 26.7 | 24.6 |
| Embodiment 21 | 28.4 | 25.6 | 57 | 0.037 | 136 | 0.40 | 6.3 | 604 | 26.3 | 24.6 |
| Embodiment 22 | 28.7 | 25.4 | 49 | 0.033 | 135 | 0.35 | 6.9 | 610 | 25.7 | 23.2 |
| Embodiment 23 | 28.3 | 25.6 | 60 | 0.036 | 134 | 0.37 | 7.2 | 608 | 25.4 | 23.4 |
| Embodiment 24 | 28.4 | 25 | 55 | 0.037 | 132 | 0.39 | 7.5 | 610 | 25.3 | 22.7 |
| Comparative example 1 | 40.0 | 14.7 | 2 | 0.021 | 350 | 0.21 | 8.5 | 611 | 2.6 | 1.8 |
| Comparative example 2 | 28.9 | 24.3 | 39 | 0.028 | 197 | 0.31 | 8.6 | 602 | 20.9 | 18.7 |
| Comparative example 3 | 28.1 | 24.9 | 38 | 0.036 | 170 | 0.38 | 5.9 | 625 | 22.9 | 24.6 |
| Comparative example 4 | - | - | - | - | - | - | - | - | - | - |
| Comparative example 5 | 28.4 | 24.5 | 35 | 0.033 | 159 | 0.32 | 6.1 | 631 | 23.9 | 26.1 |
| Comparative example 6 | 28.3 | 24.7 | 37 | 0.032 | 156 | 0.33 | 6.5 | 624 | 24.2 | 24.9 |
| Comparative example 7 | 28.1 | 24.7 | 35 | 0.033 | 169 | 0.32 | 10.8 | 611 | 22.1 | 22.6 |
| Comparative example 8 | 28.4 | 24.9 | 30 | 0.027 | 182 | 0.33 | 7.5 | 624 | 21.2 | 22.9 |

Since the second diameter of the outlet hole of the perforated plate with changeable hole diameters of comparative example 4 was undersize, the colloid gel cannot be discharged smoothly such that motor of the pulverizer was overheated. Thus, the superabsorbent polymer particles and the superabsorbent polymers could not be obtained, and various physical properties in table 2 and table 3 could not be measured.

According to table 2 and table 3, absorption properties of the superabsorbent polymers of comparative examples 1 obtained by using the conventional method are not as good as embodiments 1-24 obviously. The thickness of perforated plate of comparative example 2 was too small, such that extrusion path was too short; thus, the superabsorbent polymers with better absorption properties cannot be obtained. Relatively, the thickness of perforated plate of comparative example 3 was too great, thereby greater abrasion; thus, the surface porosity of the superabsorbent polymers would be reduced, and the absorption rate was worse. Comparative examples 7 and 8 both had the same diameters of the inlet hole and the outlet hole, such that extrusion force was insufficient, thereby resulting in higher water soluble components for the superabsorbent polymers.

According to table 2, compared to comparative examples 1 to 8, the superabsorbent polymer particles of embodiments 1 to 24 had less water soluble components and higher clean water absorption rate for 1 minute, surface porosity and free swell rate.

According to table 2, compared to comparative examples 1 to 8, embodiments 1 to 24 had greater absorption against pressure, UPM, FHA and free swell GBP and lower T20 value. Therefore, the superabsorbent polymers of embodiments 1 to 24 could absorb liquid in an easier manner under dried condition, and permeability and conductivity for liquid were better.

Therefore, with the method of producing the superabsorbent polymer of the present invention, compactness and surface roughness of the colloid gel can be increased by shearing the colloid gel by using the pulverizer having the perforated plate with changeable hole diameters. Furthermore, the bulk density, the absorption rate and the liquid conductivity of the obtained superabsorbent polymer can be increased.

**Claims**

1. A method of fabricating a superabsorbent polymer, comprising:

   performing a free radical polymerization reaction to a superabsorbent polymer composition, so as to obtain a colloid gel, wherein the superabsorbent polymer composition comprises an acid group-containing monomer aqueous solution, a polymerization initiator, and a free radical polymerization crosslinking agent;
   shearing the colloid gel by using a pulverizer to obtain a plurality of superabsorbent polymer particles, wherein the pulverizer has a perforated plate with changeable hole diameters, the perforated plate with changeable hole diameters comprises an inlet hole and an outlet hole, the inlet hole has a first diameter, the outlet hole has a second diameter, and the first diameter is greater than the second diameter; and
   performing a surface crosslinking reaction to the superabsorbent polymer particles, so as to obtain the superabsorbent polymer.

2. The method of claim 1, wherein the first diameter is in a range of 8 mm to 20 mm.

3. The method of any one of claims 1or 2, wherein the second diameter is in a range of 6 mm to 18 mm.

4. The method of any one of claims 1-3, wherein a thickness of the perforated plate with changeable hole diameters is in a range of 20 mm to 40 mm.

5. The method of claim 4, wherein the first diameter, the second diameter and the thickness of the perforated plate with changeable hole diameters have following formula:

$$\alpha = \frac{(D1 - D2)/2}{L}$$

   in the above formula, D1 represents the first diameter, D2 represents the second diameter, and L represents the thickness of the perforated plate with changeable hole diameters; and
   a value of $\alpha$ is in a range of 0.05 to 0.35.

6. The method of any one of claims 1-5, wherein shearing the colloid gel further comprises:
   screening a plurality of small colloid gels with an average diameter not greater than 2.00 mm.

7. The method of any one of claims 1-6, further comprises:
adding a surface crosslinking agent and an aluminum salt to the superabsorbent polymer particles before performing the surface crosslinking reaction.

8. The method of claim 7, wherein based on an amount of the superabsorbent polymer particles as 100 wt%, an amount of the aluminum salt is 0.1 wt% to 1.0 wt%.

9. The method of any one of claims 7 or 8, wherein the aluminum salt comprises aluminum sulfate, aluminum lactate, aluminum citrate and combinations thereof.

10. A method of fabricating a superabsorbent polymer, comprising:

performing a free radical polymerization reaction to a superabsorbent polymer composition, so as to obtain a colloid gel, wherein the superabsorbent polymer composition comprises an unsaturated monomer aqueous solution, a polymerization initiator, and a free radical polymerization crosslinking agent;
shearing the colloid gel by using a pulverizer to obtain a plurality of superabsorbent polymer particles, wherein the pulverizer has a perforated plate with changeable hole diameters, the perforated plate with changeable hole diameters comprises an inlet hole with a first diameter in a range of 8 mm to 20 mm and a outlet hole with a second diameter in a range of 6 mm to 18 mm; and
performing a surface crosslinking reaction to the superabsorbent polymer particles and a surface crosslinking agent, so as to obtain the superabsorbent polymer.

11. The method of claim 10, wherein the first diameter is greater than the second diameter.

12. The method of any one of claims 10 or 11, wherein a thickness of the perforated plate with changeable hole diameters is in a range of 20 mm to 40 mm.

13. The method of claim 12, wherein the first diameter, the second diameter and the thickness of the perforated plate with changeable hole diameters have following formula:

$$\alpha = \frac{(D1 - D2)/2}{L}$$

in the above formula, D1 represents the first diameter, D2 represents the second diameter, and L represents the thickness of the perforated plate with changeable hole diameters; and
a value of $\alpha$ is in a range of 0.05 to 0.35.

14. The method of any one of claims 10-13, further comprises:
adding an aluminum salt before performing the surface crosslinking reaction, wherein based on an amount of the superabsorbent polymer particles as 100 wt%, an amount of the aluminum salt is 0.1 wt% to 1.0 wt%.

15. The method of any one of claims 10-14, wherein based on an amount of the unsaturated monomer aqueous solution as 100 wt%, an amount of the free radical polymerization crosslinking agent is 0.001 wt% to 5 wt%.

**Amended claims in accordance with Rule 137(2) EPC.**

1. A method of fabricating a superabsorbent polymer, comprising:

performing a free radical polymerization reaction to a superabsorbent polymer composition, so as to obtain a colloid gel, wherein the superabsorbent polymer composition comprises an acid group-containing monomer aqueous solution, a polymerization initiator, and a free radical polymerization crosslinking agent;
shearing the colloid gel by using a pulverizer to obtain a plurality of superabsorbent polymer particles, wherein the pulverizer has a perforated plate with changeable hole diameters, the perforated plate with changeable hole diameters comprises an inlet hole and an outlet hole, the inlet hole has a first diameter, the outlet hole has a second diameter, and the first diameter is greater than the second diameter; wherein the first diameter is in a range of 8 mm to 20 mm and the second diameter is in a range of 6 mm to 18 mm; and

performing a surface crosslinking reaction to the superabsorbent polymer particles, so as to obtain the superabsorbent polymer.

2. The method of claim 1, wherein a thickness of the perforated plate with changeable hole diameters is in a range of 20 mm to 40 mm.

3. The method of claim 2, wherein the first diameter, the second diameter and the thickness of the perforated plate with changeable hole diameters have following formula:

$$\alpha = \frac{(D1 - D2)/2}{L}$$

in the above formula, D1 represents the first diameter, D2 represents the second diameter, and L represents the thickness of the perforated plate with changeable hole diameters; and
a value of $\alpha$ is in a range of 0.05 to 0.35.

4. The method of any one of claims 1-3, further comprises:
adding a surface crosslinking agent and an aluminum salt to the superabsorbent polymer particles before performing the surface crosslinking reaction.

5. The method of claim 4, wherein based on an amount of the superabsorbent polymer particles as 100 wt%, an amount of the aluminum salt is 0.1 wt% to 1.0 wt%.

6. The method of any one of claims 4 or 5, wherein the aluminum salt comprises aluminum sulfate, aluminum lactate, aluminum citrate and combinations thereof.

100

```
┌─────────────────────────────────────────────────────────┐
│  perform a free radical polymerization reaction to a      │
│  superabsorbent                                           │──── 110
│  polymer composition to obtain a colloid gel              │
└─────────────────────────────────────────────────────────┘
                            │
                            ▼
┌─────────────────────────────────────────────────────────┐
│  shear the colloid gel by using a pulverizer to obtain    │
│                                                           │──── 120
│  plural superabsorbent polymer particles                  │
└─────────────────────────────────────────────────────────┘
                            │
                            ▼
┌─────────────────────────────────────────────────────────┐
│  perform a surface crosslinking reaction to the           │
│  superabsorbent                                           │──── 130
│  polymer particles to obtain the superabsorbent polymer   │
└─────────────────────────────────────────────────────────┘
```

Fig. 1

Fig. 2

EP 4 582 469 A1

Europäisches
Patentamt

European
Patent Office

Office européen
des brevets

# EUROPEAN SEARCH REPORT

**Application Number**

EP 24 16 7745

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 795 616 A1 (NIPPON CATALYTIC CHEM IND [JP]) 24 March 2021 (2021-03-24) * paragraphs [0022], [0207], [0209] * ----- | 1-15 | INV. C08J3/24 C08J3/12 |
| A | US 2014/031473 A1 (NOGI KOZO [JP] ET AL) 30 January 2014 (2014-01-30) * paragraph [0749] * ----- | 1-15 | |
| A | EP 2 557 095 A1 (NIPPON CATALYTIC CHEM IND [JP]) 13 February 2013 (2013-02-13) * paragraphs [0094], [0095] * ----- | 1-15 | |

**TECHNICAL FIELDS SEARCHED (IPC)**

C08J
G01N
C08F
B01J

The present search report has been drawn up for all claims

| Place of search | Date of completion of the search | Examiner |
|---|---|---|
| Munich | 24 September 2024 | Schweissguth, Martin |

EPO FORM 1503 03.82 (P04C01)

## ANNEX TO THE EUROPEAN SEARCH REPORT
## ON EUROPEAN PATENT APPLICATION NO.

EP 24 16 7745

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2024

| Patent document cited in search report | | Publication date | Patent family member(s) | | Publication date |
|---|---|---|---|---|---|
| EP 3795616 | A1 | 24-03-2021 | CN | 112135864 A | 25-12-2020 |
| | | | EP | 3795616 A1 | 24-03-2021 |
| | | | JP | 7174756 B2 | 17-11-2022 |
| | | | JP | WO2019221154 A1 | 27-05-2021 |
| | | | KR | 20210013101 A | 03-02-2021 |
| | | | US | 2021269606 A1 | 02-09-2021 |
| | | | WO | 2019221154 A1 | 21-11-2019 |
| US 2014031473 | A1 | 30-01-2014 | CN | 103459473 A | 18-12-2013 |
| | | | EP | 2669318 A1 | 04-12-2013 |
| | | | JP | 5722921 B2 | 27-05-2015 |
| | | | JP | 6094930 B2 | 15-03-2017 |
| | | | JP | 2015120933 A | 02-07-2015 |
| | | | JP | WO2012102407 A1 | 03-07-2014 |
| | | | US | 2014031473 A1 | 30-01-2014 |
| | | | WO | 2012102407 A1 | 02-08-2012 |
| EP 2557095 | A1 | 13-02-2013 | CN | 102822209 A | 12-12-2012 |
| | | | CN | 104212105 A | 17-12-2014 |
| | | | EP | 2557095 A1 | 13-02-2013 |
| | | | EP | 3115382 A1 | 11-01-2017 |
| | | | JP | 5676572 B2 | 25-02-2015 |
| | | | JP | 6093751 B2 | 08-03-2017 |
| | | | JP | 2015083693 A | 30-04-2015 |
| | | | JP | WO2011126079 A1 | 11-07-2013 |
| | | | KR | 20130093477 A | 22-08-2013 |
| | | | KR | 20180112110 A | 11-10-2018 |
| | | | US | 2013026412 A1 | 31-01-2013 |
| | | | US | 2016332141 A1 | 17-11-2016 |
| | | | WO | 2011126079 A1 | 13-10-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1206365 A
- WO 2012174026 A1
- US 9285302 B
- US 7108916 B
- US 8021998 B2